(19) Europäisches Patentamt
European Patent Office
Office européen des brevets

(11) **EP 4 631 419 A1**

(12) **EUROPEAN PATENT APPLICATION**

(43) Date of publication:
**15.10.2025 Bulletin 2025/42**

(21) Application number: **25168859.4**

(22) Date of filing: **07.04.2025**

(51) International Patent Classification (IPC):
**A61B 5/00** (2006.01)   **G16H 50/20** (2018.01)
**G16H 50/30** (2018.01)

(52) Cooperative Patent Classification (CPC):
**A61B 5/7275; A61B 5/4064; A61B 5/4362;
G16H 50/20; G16H 50/30;** A61B 2503/02;
A61B 2503/045

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **08.04.2024 EP 24169024**

(71) Applicant: **Jensen, Arne
44797 Bochum (DE)**

(72) Inventor: **Jensen, Arne
44797 Bochum (DE)**

(74) Representative: **Uexküll & Stolberg
Partnerschaft von
Patent- und Rechtsanwälten mbB
Beselerstraße 4
22607 Hamburg (DE)**

(54) **PREDICTING A RISK OF BRAIN DAMAGE IN A NEWBORN AND/OR A PHYSICAL STATE OF THE NEWBORN PRIOR TO BIRTH OF THE NEWBORN DURING PREGNANCY**

(57)    A method for predicting a risk of brain damage in a newborn and/or a physical state of the newborn prior to birth of the newborn during pregnancy comprises the following steps:
i) Determining the presence of one or more risk factors during pregnancy which are associated with brain damage in the newborn;
ii) Calculating a pregnancy risk score by summing up odds ratios which quantify the association between the one or more risk factors and a poor predicted psychomotor development of infants at preschool age;
iii) Using a regression equation to predict a risk of brain damage in the newborn and/or the physical state of the newborn based on the calculated pregnancy risk score.

Fig. 2

EP 4 631 419 A1

**Description**

FIELD OF INVENTION

The present invention relates to a method for predicting a risk for brain damage in a newborn and/or a physical state of the newborn prior to birth of the newborn during pregnancy as a fetus.

The brain is a complex organ and has a sensitive response to external influences. In contrast to the heart, liver or lung, it does not survive oxygen deprivation for more than eight to ten minutes. In addition, it is particularly sensitive to inflammatory reactions, trauma and genetic aberrations.

The causes of brain damage in newborns and preterm infants are numerous and range from placental circulatory disturbances, clamped vessels during birth, infections, traumatic events during birth and genetic defects to consequences of artificial ventilation in very immature preterm infants. In Germany, around 1000 children suffer very severe brain damage each year as a direct result of oxygen deprivation immediately before, during or after birth. Newborns with a birth weight of lower than 1500 grams are at particular risk. Depending on the extent of the damage and the brain regions affected, they can suffer minor dysfunctions of the brain (e.g. "minimal brain dysfunction", "attention deficit hyperactivity disorder") or very severe physical and extensive mental disabilities. These include movement disorders up to arm and leg paralysis, and today even an association with schizophrenia is suspected. The control of emotional impulses can also be impaired, which in adult age can lead to lower mental performance, relationship problems, anxiety, depression or difficulties in adjusting to work.

The burden on those affected and their relatives is considerable and close cooperation between obstetricians, paediatricians, psychologists and speech and movement therapists is needed. The costs that arise for the German collective insurance body are estimated to be at least € 500 million for each birth year.

In very preterm infants oxygen deprivation during birth usually damages the white brain matter surrounding the natural hollow spaces (ventricles) inside the brain. As a result of this so-called periventricular leukomalacia, nerves running from the cerebral cortex, inter alia, to the legs are damaged, which causes the mentioned movement disorders. In addition, the sensitive blood vessels of the immature brain can easily tear upon blood pressure fluctuations associated with oxygen deprivation. This results in bleeding into the cerebral fluid or into the white brain matter.

In contrast, in full-term infants, oxygen deprivation leads more to damage in the gray matter of the cerebral cortex, diencephalon and mesencephalon. However, this affects at most 0.04 % of infants, while severe brain damage occurs in 10 to 15% of all preterm infants with a birth weight of less than 1500 grams.

Some clinical strategies aimed at protecting infant brain cells and in particular at preventing brain damage in preterm infants have shown good results over the past ten years. Reducing the rate of preterm deliveries is in the foreground, but also early recognition of oxygen deprivation and the appropriate interventions.

Stress situations for the mother and child, bleeding and infections that spread into the uterus increase the risk of preterm delivery. A comprehensive medical history is therefore first gathered in order to recognize risk patients in time.

A comprehensive ultrasound mass screening of infant brains after birth (approximately 5300 children) revealed that all grades of cranial haemorrhages (I to IV) increase with decreasing vitality, in particular in preterm infants (see Berger et al., Eur. J. Obstet. Gynaecol. Reprod. Biol. 75 (1997) 191-203). The so-called Apgar score is used as a measure of vitality, which takes into account children's heart rate, respiratory effort, muscle tone, skin coloration and response to stimulation (0 to 10 points). It is measured at one, five and ten minutes after every birth and is between eight and ten points in healthy children. A low Apgar score may reflect an oxygen deprivation-induced shock state in the child. While lack of oxygen in the brain usually causes the sympathetic nervous system (part of the autonomic nervous system) to increasingly direct blood flow to vital organs from other regions of the organism that are less vulnerable, preterm infants have only a limited capacity for this kind of redirection. This, together with the sensitivity of their blood vessels to blood pressure fluctuations, leads to an extremely low tolerance for oxygen deprivation.

These results led to the concept of early intervention: At the first signs of potential oxygen deprivation, a risk assessment is performed, and all preparations made so that the oxygen-deprived infant (decreasing heart rate) is born immediately and under ideal conditions. If successful, an infant in a good state can be further monitored by the paediatrician and damage to the immature brain prevented.

As brain damage cannot always be prevented by screening or early intervention, therapeutic strategies are also being researched to protect and even regenerate the infant brain after damage has already occurred. This requires that the mechanisms leading to brain damage be known.

It was determined that death of oxygen-deprived nerve cells in the brain begins below a threshold value and then proceeds in two waves: The lack of circulation causes the energy metabolism in the brain to collapse, and in response to the lack of oxygen increased amounts of glutamate are released - one of the most important activating messengers in the brain. If energy is no longer available for ion exchange across the cell membrane, the electrical potential across the membrane of nerve cells cannot be maintained either. As a result, large amounts of calcium enter the cells through various ion channels. Some of these channels are regulated by glutamate. The excessive rise in the intracellular calcium concentration, the so-called calcium overload, activates various enzymes that ultimately damage the cells.

Initially, once the acute lack of oxygen is over, the energy metabolism normalizes in several regions of the brain. However, a few hours later a second wave of nerve cell death begins: The nerve cells swell, epileptic activity patterns are measured in the brainwaves, indicating damage to the nerve cells. The reasons for this are thought to include inflammatory reactions and an imbalance between inhibiting and activating messengers in the brain that might trigger programmed cell death (apoptosis). Attempts at intervention are made within this "therapeutic window", as a significant number of cells are damaged only hours or days following oxygen deprivation.

Various drug-based strategies are already being investigated that are able to alleviate brain damage caused by lack of oxygen, for example, neuroprotective substances, i.e. substances that protect nerve cells. For example, flunarizine is used, which reduces the uncontrolled influx of calcium into oxygen-deprived nerve cells (calcium channel antagonist), and lubeluzole, a glutamate antagonist. Both substances had already shown positive results in stroke. While lubeluzole did not have the desired effect in newborns, flunarizine appears suitable for protecting the infant brain from severe damage resulting from oxygen deprivation.

As any pharmaceutical introduced into obstetrics requires extensive safety evaluations, a search was concurrently made for different substances endogenous to the body that have a similarly beneficial effect. Promising results are already being observed with the energy-rich compound creatine, a metabolic product of the organism (R. Berger et al., Creatine protects the immature brain from hypoxic-ischemic injury, Middelanis et al., 2003, J. Soc. Gynecol. Investig. Vol. 10, No: 2, Abstract No. 290).

Another approach is treatment of brain damage in preterm and full-term infants with cord blood to achieve functional neuroregeneration (A. Jensen, "Stammzellen aus Nabelschnurblut heilen kindlichen Hirnschaden" [Stem cells from cord blood heal infant brain damage] Top Magazine Ruhr, Wissenschaft - Medizin [Science - Medicine] 23(4), 80-81 (2009); A. Jensen, "Erste Therapie eines kindlichen hypoxischen Hirnschadens mit Zerebralparese nach Herzstillstand? [First therapy of hypoxic infant brain damage with cerebral paresis after cardiac arrest?] - Heilversuch durch autologe Nabelschnurstammzell-Transplantation." [attempt at healing through autologous cord stem cell transplantation] Regenerative Medizin [Medicine] 4(1), 30-31 (2011)). The publications by A. Jensen et al. "Perinatal brain damage - from neuroprotection to neuro regeneration using cord blood stem cells" Med. Klein. 98 (2003) Suppl. 2, pages 22-26 and C. Meier et al. "Spastic paresis after perinatal brain damage in rats is reduced by human cord blood mononuclear cells" Pediatr. Res. 2006; 59(2), pages 244-249 were able to show that systemic transplantation of human mononuclear cells from cord blood into newborn rats with experimental brain damage led both to a massive migration of these cells into the damaged brain region and prevention of spastic pareses. Spasticity, the leading symptom of infant cerebral paresis, was practically no longer detectable in the transplanted rats.

It would, however, be ideal to treat brain-damaged preterm and full-term infants primarily with fresh, and not cryopreserved, autologous cord blood. However, for this purpose, the decision to store the umbilical cord blood for later treatment of the child's brain damage can only be made before birth.

An object of the present invention is therefore to provide a method for predicting a risk of brain damage in a newborn or a physical state of the newborn prior to birth of the newborn during pregnancy.

SUMMARY OF THE INVENTION

The object is achieved with a method as defined in present claim 1. Preferred embodiments are set out in the appending sub-claims.

According to the invention, there is provided a method for predicting a risk of brain damage in a newborn and/or a physical state of the newborn prior to birth of the newborn during pregnancy, the method comprising the following steps:

i) Determining the presence of one or more risk factors during pregnancy which are associated with brain damage in the newborn;

ii) Calculating a pregnancy risk score by summing up odds ratios which quantify the association between the one or more risk factors and a poor predicted psychomotor development of infants at preschool age;

iii) Using a regression equation to predict a risk of brain damage in the newborn and/or the physical state of the newborn based on the calculated pregnancy risk score.

Preferably, the method provides a clinical diagnostic support.

The invention will now be further described with reference to the appended Tables and Figures in which:

Table 1   shows risk factors and odds ratios which may be used in steps i) and ii), respectively,

Table 2   shows values of a predicted Intelligence quotient (pIQ), a Maze test (pMT) and a Neurologic examination optimality score (pNOS),

Figure 1   shows the interrelation between the predicted brain damage and pregnancy risk score,

Figure 2 shows the ROC-analysis result of predicted brain damage vs. pregnancy risk score,
Figure 3 shows the interrelation between the predicted Apar score at 1 minute and the pregnancy risk score,
Figure 4 shows the ROC-analysis result of predicted Apgar score at 1 minute vs. pregnancy risk score,
Figure 5 shows the interrelation between the predicted Apgar score at 5 minutes and the pregnancy risk score,
Figure 6 shows the ROC-analysis result of predicted Apgar score at 5 minutes vs. pregnancy risk score,
Figure 7 shows the interrelation between the predicted Apgar score at 10 minutes and the pregnancy risk score,
Figure 8 shows the ROC-analysis result of predicted Apgar score at 10 minutes vs. pregnancy risk score.

A list of risk factors which may be determined in step i) are shown in Table 1. Table 1 also lists odds ratios, which are assigned to the corresponding risk factors and which are used in step ii) in case one or more of the corresponding risk factors have been determined in step i). The risk factors are listed under the heading "variables" in the first left column. The odds ratios are listed under the heading "Odds ratios" in the third left column relating to the predicted Psychomotor Development pTPMDS.

The odds ratios as shown in Table 1 were calculated for composite psychomotor development indices (pTPMDS, pDDI) based on predicted (p) Intelligence quotient (pIQ), Maze test (pMT), Neurologic examination optimality score (pNOS), and cMVI based on growth variables and Apgar Score at 10 mins as shown below in Table 2. Odds ratios quantify the association between the one or more risk factors and a poor predicted psychomotor development at preschool age. In this connection reference is made to the publication of A. Jensen et. al. "Growth variables and obstetrical risk factors in newborns are associated with psychomotor development at preschool age", AJOG Global Reports 2023; May 8;3(4):100219, which shows that morphometric measures from newborns at birth like weight/head circumference ratio predict overall psychomotor development at 4.3 years (standard deviation, 0.8) of preschool age. Moreover, the study shows that neonatal cerebral hemorrhage and white matter damage (brain damage) condition psychomotor development and educational success during childhood and adolescence and further shows the calculation and use of odds ratios. In connection with the present invention the term "poor predicted psychomotor development" is associated with the presence of reduced Apgar scores, i.e. below 7, measured at the newborn at five and ten minutes after birth.

The pregnancy risk score as used in step ii) above is the sum of the one or more odds ratios which are assigned to the corresponding risk factors which are determined in step i) above.

In the list of risks factors as defined in Table 1, the term 'Breech presentation' should read 'Presentation' (position of the child in the womb). In general, the position of the child is coded in the database as follows: 1 = Vertex presentation (cranial position), 2 = Oblique presentation (transverse position), 3 = Breech presentation (pelvic presentation). In table 1 as mentioned below the term 'Breech presentation' is mentioned. However, this is only a special case of the 'Presentation' category.

The method according to the present invention provides for the first time a reliable prediction of brain damage and the physical state of the newborn, e.g. the Apgar scores of the newborn, before birth. The prediction of brain damage before birth is important clinically to enhance risk stratification for a timely gentle and safe delivery and provides the opportunity for precautionary cord blood banking as a basis for potential stem cell treatments in the newborn period to ameliorate brain damage.

Furthermore, the odds ratios used in combination with the method according to the present invention also take into account microstructural changes of the newborn brain that are not recognizable on ultrasound, such as those occurring in children with intrauterine growth retardation (IUGR).

In an embodiment of the present invention the one or more risk factors which are used in step i) above are chosen from a list consisting of gestational age, brain body weight ratio, weight/head circumference ratio, preterm birth ≤36 wk, weight/length ratio, IUGR (intrauterine growth restriction), multiples (twins), Apgar 1 min, score <9, Apgar 1 min, score <7, Apgar 5 min, score <10, Apgar 5 min. score <9, Apgar 10 min. score <10, Apgar 10 min, score <9, pH umbilical artery <7.29 vs. ≥7.29, PIVH grade 1+2, PIVH grade 3, PIVH grade 4, PIVH present (all grades), WMD present, PIVH plus WMD vs PIVH only, PIVH without WMD, PIVH grade 1+2 (exclusive), presentation, vaginal delivery, cardiotocography pathologic, sex, amnion infection, vaginal bleeding, hypertension, prolonged or arrested labor, primiparity, maternal age <3% centile, transfer to NICU, malformation, meconium stained amniotic fluid, PROM (premature rupture of membranes), EPH syndrome (edema, proteinuria, hypertension during pregnancy), miscarriage (miscarriages in the past, medical history), maternal fever >38°C (during delivery), Rh incompatibility (Rhesus factor incompatibility between mother and child), diabetes mellitus of the mother, maternal age >97% centile, hypotension. More preferably, only those risk factors are chosen from the list shown in Table 1 above which can be examined during pregnancy at the fetus and/or its mother.

In order to determine the predictive capacity of the risk factors on brain damage in a newborn a prospective cranial ultrasound screening (CUS) study (1984-1988) was used as a basis which was carried out on 5,301 live-born infants, including 571 (10.8%) preterms (≤36 weeks), on the day of discharge of the mother at 5-8 days postpartum (after excluding those 498 [8.6%] that left early, i.e., at ≤4 days) from a level III perinatal center at Giessen University, Germany. All neonates (51.0% male) underwent CUS to detect cerebral hemorrhage and/or white matter damage, i.e. brain damage. Furthermore, in a previous study (1982-86) from the same center, both cranial ultrasound screening results after birth and psychomotor development (PMD) were determined in 137 (2.4%) children at 4.3 (standard deviation [SD], 0.8) years preschool age in a matched pair design, strictly controlling for confounders, for example, sex, socioeconomic status, maternal education, and brain damage. Intelligence quotient (IQ), Maze test (MT; adapted by Kramer et al, 1985),15 and Neurologic examination optimality score (NOS) were measured (m) and an average composite Total psychomotor development score (mTPMDS) for overall psychomotor development was formed (mTPMDS=[zIntelligence quotient IQ+zMaze test result+zNeurologic examination optimality score]/3). These psychomotor development data were extrapolated to the whole ultrasound screening cohort (n=5,301) as follows: The measured psychomotor development testing results as assessed by the Total psychomotor development score were used to generate a prediction model with measured Total psychomotor development score as dependent variable by stepwise multiple regression analysis (pTPMDS = 17.87 + 0.00043 * weight 0.501 * WMD_present + 2.278 * Ph_umb.art + 0.177 * mode of delivery (r=0.637, n=129, P<.001)) that correlated well with the measured results (r=0.598, n=130, P<.001) and hence was used for extrapolation (n=5,301).

Moreover, using a stepwise multiple regression analysis the risk factors having an impact on the documented neonatal brain damage, i.e., cerebral hemorrhage and/or white matter damage, as a dependent variable and the extent of the impact, the predictors, was defined. All the risk factors as shown in table 1 and/or defined thereafter are included as independent variables in the calculation of the dependent variable brain damage (cerebral hemorrhage and/or brain damage in the white matter). The multiple regression analysis resulted in the following multiple regression equation:

Brain damage = 1.38 - 0.034 * gestational age + 0.038 * multiples + 0.022 * cardiotocogram pathologic + 0.264 * amnion infection + 0.065 * maternal fever > 38°C + 0.018 * prolonged or arrested labor + 0.135 * malformation (r = 0.426, n = 5,095, P < 0.001).

Thus, based on the above regression equation it has been found in combination with the present invention and based on the above mentioned cranial ultrasound screening (CUS) study (1984-1988) that the risk factors as shown in table 1 and in particular the following risk factors bear predictive capacity for brain damage in newborn infants before birth, namely gestational age, multiples, cardiotocogram pathologic, amnion infection, maternal fever>38°C, prolonged or arrested labor and malformation.

In another embodiment of the present invention the regression equation in step iii) is a linear regression equation which contains a constant and a regression coefficient, wherein the constant and the regression coefficient have been calculated using a linear regression analysis.

In connection with the present invention, a pregnancy risk score based on odds ratios was used to better quantify the adverse effects of risk factors on brain damage. Furthermore, using the odds ratios in combination with the method according to the present invention also take into account microstructural changes of the newborn brain that are not recognizable on ultrasound, such as those occurring in children with intrauterine growth retardation (IUGR).

The odds ratios (probability factor) of poor predicted psychomotor development (predicted Total Psychomotor Development Score, pTPMDS) in the 4th year of life of the children were assigned to the risk factors as listed in table 1 above. Each pregnancy of the 5,301 patients documented in the CUS study (1984-1988) was analyzed with regard to the presence of one or more of the risk factors. A pregnancy risk score was calculated for each of the pregnancies of the 5,301 patients documented in the CUS study (1984-1988) by summing up the odds ratios assigned to the one or mor risk factors present in the individual pregnancy.

Using linear regression analysis, the constant as well as the regression coefficient of the corresponding linear regression equation were calculated. Thus, the interrelation of the pregnancy risk score and brain damage of the newborns included in the CUS study (1984-1988) was determined.

Preferably, in step iii) of the method according to the present invention the following linear regression equation is used to calculate the risk of brain damage as a dependent variable:

$$pBrain\_damage = b_1 + m_1 * \text{pregnancy risk score,}$$

wherein $b_1$ is chosen from the range of 0.0 to 0.1, preferably of 0.0 to 0.05, more preferably 0.021 and, wherein $m_1$ is chosen from the range of 0.0 to 0.1, preferably 0.0 to 0.01, more preferably 0.005.

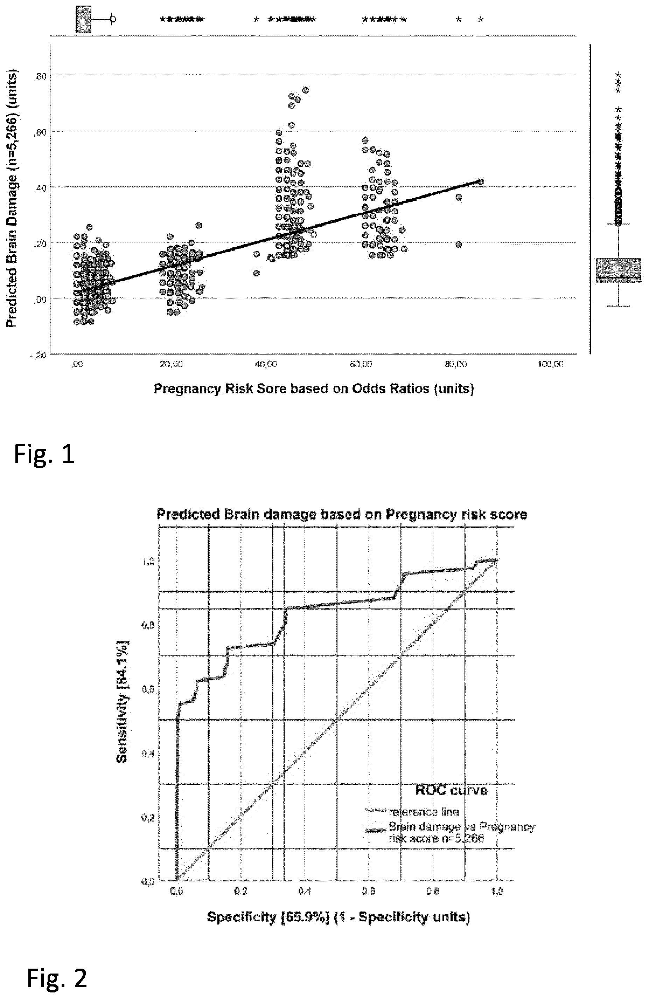

Figure 1 visualizes the linear regression equation as defined above (black solid line) and thus the interrelation between the pregnancy risk score and the predicted brain damage.

The linear regression equation which has been determined based on the CUS study (1984-1988) using linear regression analysis is capable to predict the risk of brain damage in any newborn based on the risk factors determined during pregnancy at the corresponding mother and/or the fetus (see step i) and the odds ratios odds ratios which quantify the association between the one or more risk factors and a poor predicted psychomotor development of infants at preschool age (see step ii). The odds ratios may be stored in a database which is accessible while calculating the pregnancy risk score in order to retrieve the odds ratios therefrom (see step ii).

Figure 2 shows the results of a Receiver operating characteristics analysis (ROC-analysis) of Predicted brain damage vs. Pregnancy risk score. The results are depicted as a function with an approximately arched shape. Figure 2 also shows a reference line depicted as a straight line with a constant gradient. The ROC-analysis revealed a sensitivity of 84.1% at a specificity of 65.9% (n=5,266, P>0.001), and a positive predictive value (PPV) of 79.4% and negative predictive value (NPV) of 72.3%, respectively (see Figure 2 below). Thus, brain damage can be predicted before birth with substantial certainty using pregnancy risk scores as a solid basis for the decision to bank cord blood for potential stem cell treatment in the newborn to improve neurocognition. Moreover, brain damage can be predicted as a solid basis for preventive strategies both to ensure unimpaired vitality of the newborn and to improve psychomotor development in childhood.

In an embodiment of the present invention the physical state of the newborn is defined by an Apgar score at 1 minute (Apgar 1 min), an Apgar score at 5 minutes (Apgar 5 min) and/or an Apgar score at 10 minutes (Apgar 10 min) after birth of the newborn and, wherein in step iii) the following linear regression equation is used to calculate Apgar 1 min, Apgar 5 min and/or Apgar 10 min:

a) $pApgar\_1 = b_2 - m_2 *$ pregnancy risk score, wherein pApgar_1 is the predicted Apgar 1 min, $b_2$ is chosen from the range of 8.5 to 9, preferably 8.75 to 8.9, more preferably 8.889 and wherein $m_2$ is chosen from the range of 0.0 to 0.1, preferably 0.0 to 0.05, more preferably 0.041,

b) $pApgar\_5 = b_3 - m_3 *$ pregnancy risk score, wherein pApgar_5 is the predicted Apgar 5 min, $b_3$ is chosen from the range of 9.5 to 10, preferably 9.7 to 9.9, more preferably 9.858 and wherein $m_3$ is chosen from the range of 0.0 to 0.1, preferably 0.0 to 0.05, more preferably 0.024,

c) $pApgar\_10 = b_4 - m_4 *$ pregnancy risk score, wherein pApgar_10 is the predicted Apgar 10 min, $b_4$ is chosen from the range of 9.5 to 10, preferably 9.7 to 9.9, more preferably 9.980 and wherein $m_4$ is chosen from the range of 0.0 to 0.1, preferably 0.0 to 0.05, more preferably 0.014.

Signs of circulatory centralization in newborns after birth as reflected by reduced Apgar scores at 1, 5 and 10 minutes are associated with poor neurocognition in childhood and beyond. Therefore, predictive risk analyses before birth may enhance risk stratification and obstetrical management to improve clinical outcome and hence psychomotor development of the newborn child.

In order to determine the predictive capacity of the risk factors on the Apgar scores at 1, 5 and 10 minutes in a newborn the same prospective cranial ultrasound screening (CUS) study (1984-1988) was used as mentioned above with regard to the impact of the risk factors on brain damage.

Using a stepwise multiple regression analysis the risk factors having an impact on the documented Apgar scores at 1, 5 and 10 minutes in a newborn, as dependent variables and the extent of the impact, the predictors, was defined. All the risk factors as shown in table 1 and/or defined thereafter are included as independent variables in the calculation of the dependent variables Apgar scores at 1, 5 and 10 minutes in a newborn. The multiple regression analysis resulted in the following multiple regression equations:

Apgar_1 = 1.556 + 0.199 * gestational age - 0.793 * cardiotocogram pathologic - 0.376 * presentation - 1.179 * malformation - 0.344 * EPH syndrome - 0.240 * prolonged or arrested labor - 0.362 * intrauterine growth restriction - 0.299 * multiples - 0.176 * bleeding vaginal - 0.373 * diabetes mellitus - 0.925 * amnion infection - 0.060 * sex - 0.137 * stained amniotic fluid (r = 0.589, n = 5080, P < 0.001).

Apgar_5 = 4.43 + 0.142 * gestational age - 0.426 * cardiotocogram pathologic- 0.181 * presentation - 0.679 * malformation - 0.232 * growth restriction - 0.166 * multiples - 0.146 * EPH syndrome - 0.103 * prolonged or arrested labor - 0.087 * bleeding vaginal + 0.066 * premature rupture of membranes (r = 0.567, n = 5,080, P < 0.001).

Apgar_10 = 6.263 + 0.095 * gestational age - 0.214 * cardiotocogram pathologic- 0.580 * malformation - 0.062 * presentation - 0.077 * growth restriction - 0.061 * stained amniotic fluid + 0.031 * premature rupture of membranes (r = 0.552, n = 5,076, P < 0.001).

In connection with the present invention, the pregnancy risk score based on odds ratios was also used to better quantify the adverse effects of risk factors on Apgar scores at 1, 5 and 10 minutes in a newborn. The odds ratios (probability factor) of poor predicted psychomotor development (predicted Total Psychomotor Development Score, pTPMDS) in the 5th year of life of the children were assigned to the risk factors as listed in table 1 above. Each pregnancy of the 5,301 patients documented in the CUS study (1984-1988) was analyzed with regard to the presence of one or more of the risk factors. A pregnancy risk score was calculated for each of the pregnancies of the 5,301 patients documented in the CUS study (1984-1988) by summing up the odds ratios assigned to the one or mor risk factors present in the individual pregnancy.

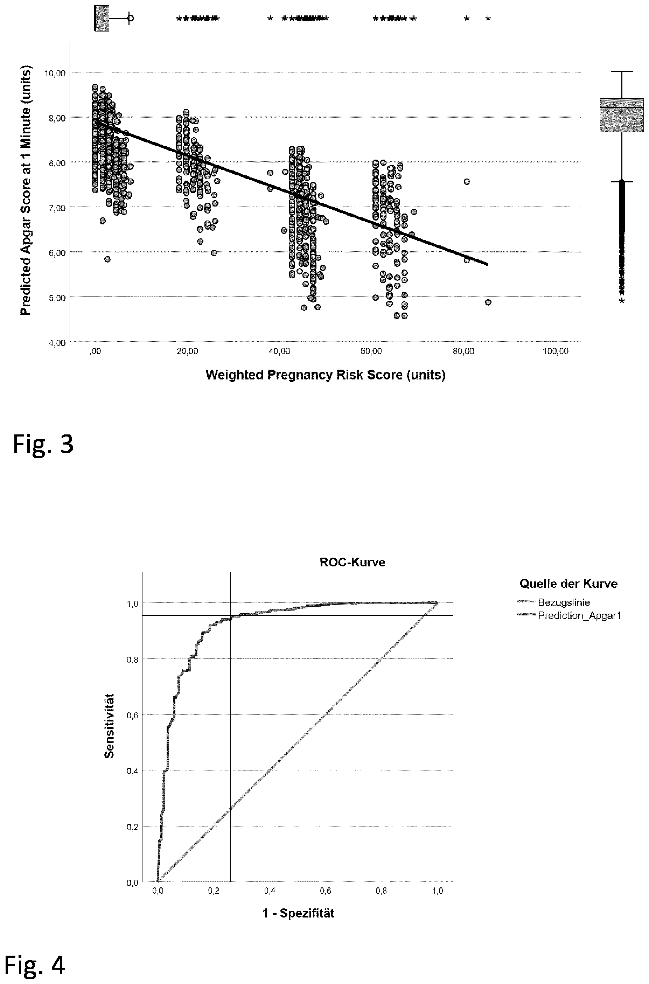

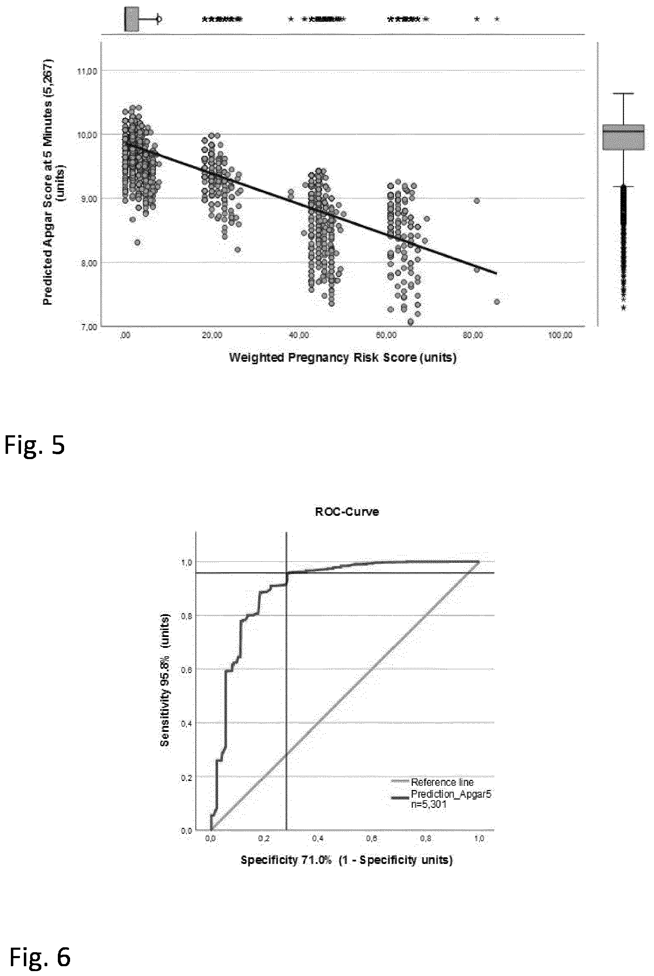

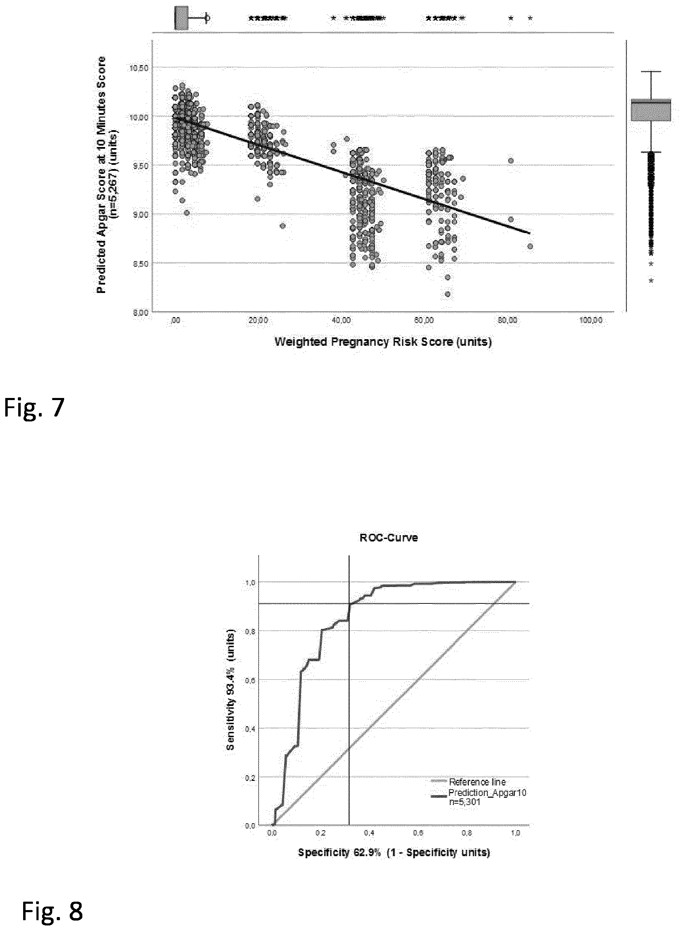

Using linear regression analysis, the constant as well as the regression coefficient of the linear regression equation corresponding to the Apgar score at 1, 5 or 10 minutes in a newborn were calculated. Thus, the interrelation of the pregnancy risk score and Apgar score at 1, 5 or 10 minutes in a newborn was determined based on the CUS study (1984-1988). The corresponding linear regression equations are given under items a) to c) above and visualized as black solid line in Figures 3, 5 and 7, wherein Figure 3 shows the regression equations with the Apgar score at 1 minute as the dependent variable, Figure 5 shows the regression equations with the Apgar score at 5 minutes as the dependent variable and Figure 7 shows the regression equations with the Apgar score at 10 minutes as the dependent variable.

The corresponding results of a Receiver operating characteristics analysis (ROC-analysis) of the predicted Apgar score at 1, 5, and 10 minute vs. pregnancy risk score are depicted in Figures 4, 6 and 8.

Figure 4 shows the results of the Receiver operating characteristics analysis (ROC-analysis) of predicted Apgar score at 1 minute vs. pregnancy risk score. The results are depicted as a function with an approximately arched shape. Figure 4 also shows a reference line depicted as a straight line with a constant gradient. The ROC-analysis resulted in a sensitivity (accuracy) of 95.1% with a specificity of 73.6% (see Figure 4 below). Thus, the neonatal Apgar score at 1 minute can be predicted before birth with substantial certainty using weighted pregnancy risk scores as a solid basis for preventive strategies both to ensure unimpaired vitality of the newborn and to improve psychomotor development in childhood.

Figure 6 shows the results of the Receiver operating characteristics analysis (ROC-analysis) of predicted Apgar score at 5 minutes vs. pregnancy risk score. The results are depicted as a function with an approximately arched shape. Figure 6 also shows a reference line depicted as a straight line with a constant gradient. The ROC-analysis revealed a sensitivity of 95.8% at a specificity of 71.0% (AUC 0.901, n=5,301, P<0.001), a positive predictive value (PPV) of 89.7% and a negative predictive value (NPV) of 64.5% (n=5,301, P>0.001) (see Figure 6 below). Thus, the neonatal Apgar score at 5 minutes can be predicted before birth with substantial certainty using weighted pregnancy risk scores as a solid basis for preventive strategies both to ensure unimpaired vitality of the newborn and to improve psychomotor development in childhood.

Figure 8 shows the results of the Receiver operating characteristics analysis (ROC-analysis) of predicted Apgar score at 10 minutes vs. pregnancy risk score. The results are depicted as a function with an approximately arched shape. Figure 8 also shows a reference line depicted as a straight line with a constant gradient. The ROC-analysis revealed a sensitivity of 93.4% at a specificity of 62.9% (AUC 0.851, n=5,301, P<0.001) (see Figure 8 below). The positive predictive value (PPV) and the negative predicted value (NPV) were 88.2% and 63.2%, respectively. Thus, neonatal Apgar score at 10 minutes can be predicted before birth with substantial certainty using weighted pregnancy risk scores as a solid basis for preventive strategies both to ensure unimpaired vitality of the newborn and to improve psychomotor development in childhood.

In another embodiment of the invention, the risk of brain damage in the newborn and/or the physical state of the newborn based on the calculated pregnancy risk score as determined in step iii) are outputted on a display. The display may be for example a computer display or the display of a smartphone.

Preferably, at least steps ii) and iii) are executed on a computer comprising a processor, a storage medium and an input interface to receive user inputs. Optionally, the computer is formed as a smartphone. Preferably, at least steps ii) and iii) of the method according to the present invention are implemented in a smartphone app which is stored on the storage medium and may be used by a pregnant woman as a user during her pregnancy. A database containing the odds ratios and/or the risk factors may be stored on the storage medium or may be wirelessly accessible.

The user may manually determine the presence of one or more risk factors during her pregnancy which are associated with brain damage in the newborn. The smartphone app may display possible risk factors and the user may confirm the presence of one or more risk factors with the app using the input interface. The processor of the smartphone may calculate thereafter a pregnancy risk score by summing up odds ratios which quantify the association between the one or more risk factors and a poor predicted psychomotor development. The processor may retrieve the odds ratios from a database stored on the storage medium of the smartphone or wirelessly access the database. The processor may further predict a risk of brain damage in the newborn and/or the physical state of the newborn using a regression equation based on the calculated pregnancy risk score. The calculated risk of brain damage may optionally outputted on the display of the smartphone.

The method according to the present invention allows a first risk assessment regarding possible brain damage of the newborn during pregnancy. Based on this risk assessment a recommendation to bank Cord blood before birth may be given. Precautionary Cord blood banking forms a basis to treat neonatal brain injury. Moreover, based on the risk assessment the psychomotor development of the offspring at 4 years of age may be predicted. The risk assessment may also be used as a basis for early intervention if child's predicted development is sub-optimal.

Table 1

**TABLE 1**

**Odds ratios and 95% confidence intervals of *predicted* Total psychomotor development score (*p*TPMDS), *calculated* Morphometric vitality index (*c*MVI), and *predicted* Developmental disability index (*p*DDI) for obstetrical risk factors in 5,301 newborns (24-43 weeks gestation, 350-5,370 g birthweight) derived from a cranial ultrasound screening data base[1,2,12]**

| Variable | Psychomotor Development (*p*TPMDS) 95% confidence interval | | | | | Morphometric Vitality Index (*c*MVI) 95% confidence interval | | | | | Developmental Disability Index (*p*DDI) 95% confidence interval | | | | |
|---|---|---|---|---|---|---|---|---|---|---|---|---|---|---|---|
| | N | Odds ratio | Lower limit | Upper limit | *P* value | N | Odds ratio | Lower limit | Upper limit | *P* value | N | Odds ratio | Lower limit | Upper limit | *P* value |
| Brain body weight ratio | 5,202 | 48.88 | 41.47 | 57.60 | .000 | 5,281 | 44.42 | 37.85 | 52.14 | .000 | 5,196 | 2.98 | 11.37 | 14.81 | .000 |
| Weight/Head circumference ratio | 5,202 | 48.87 | 41.47 | 57.60 | .000 | 5,281 | 44.72 | 38.09 | 52.50 | .000 | 5,196 | 13.04 | 11.42 | 14.88 | .000 |
| Preterm birth ≤36 wk | 5,202 | 42.73 | 25.90 | 70.48 | .000 | 5,281 | 116.70 | 52.10 | 261.42 | .000 | 5,198 | 13.86 | 10.16 | 18.90 | .000 |
| Weight/length ratio | 5,202 | 26.80 | 23.12 | 31.07 | .000 | 5,281 | 55.24 | 46.73 | 65.30 | .000 | 5,193 | 12.18 | 10.68 | 13.88 | .000 |
| IUGR | 5,202 | 19.78 | 10.15 | 38.55 | .000 | 5,281 | 187.79 | 26.33 | 1,339.08 | .000 | 5,202 | 17.70 | 9.38 | 33.39 | .000 |
| Multiples | 5,202 | 18.23 | 10.46 | 31.78 | .000 | 5,281 | 30.22 | 14.97 | 60.98 | .000 | 5,198 | 6.29 | 4.40 | 9.00 | .000 |
| Apgar 1 min, score < 9 | 5,195 | 3.57 | 3.11 | 4.10 | .000 | 5,280 | 2.82 | 2.47 | 3.23 | .000 | 5,197 | 2.39 | 2.09 | 2.73 | .000 |
| Apgar 1 min, score < 7 | 5,195 | 12.39 | 8.26 | 18.58 | .000 | 5,280 | 13.69 | 9.00 | 20.83 | .000 | 5,197 | 9.42 | 6.54 | 13.57 | .000 |
| Apgar 5 min, score < 10 | 5,194 | 4.67 | 3.95 | 5.51 | .000 | 5,278 | 4.42 | 3.75 | 5.20 | .000 | 5,195 | 3.51 | 3.00 | 4.11 | .000 |
| Apgar 5 min. score < 9 | 5,194 | 9.49 | 6.91 | 13.04 | .000 | 5,278 | 9.63 | 7.01 | 13.23 | .000 | 5,195 | 6.98 | 5.25 | 9.29 | 000 |
| Apgar 10 min. score < 10 | 5,191 | 11.01 | 8.05 | 15.05 | .000 | 5,281 | 24.62 | 15.99 | 37.91 | .000 | 5,198 | 13.40 | 9.57 | 18.76 | .000 |
| Apgar 10 min, score < 9 | 5,191 | 30.14 | 13.33 | 68.17 | .000 | 5,281 | 191.72 | 26.84 | 1,369.53 | .000 | 5,198 | 93.75 | 23.24 | 378.22 | .000 |
| pH umbilical artery <7.29 vs. ≥7.29 | 5,202 | 2.49 | 2.22 | 2.78 | .000 | 5,192 | 0.96 | 0.86 | 1.07 | .454 | 5,198 | 2.90 | 2.59 | 3.24 | .000 |
| PIVH grade 1+2 | 5,202 | 9.42 | 5.37 | 15.47 | .000 | 5,280 | 6.45 | 4.21 | 9.89 | .000 | 5,197 | | 4.28 | 10.21 | .000 |

EP 4 631 419 A1

**TABLE 1**

Odds ratios and 95% confidence intervals of *predicted* Total psychomotor development score (*p*TPMDS), *calculated* Morphometric vitality index (*c*MVI), and *predicted* Developmental disability index (*p*DDI) for obstetrical risk factors in 5,301 newborns (24-43 weeks gestation, 350-5,370 g birthweight) derived from a cranial ultrasound screening data base[1,2,12]

| Variable | Psychomotor Development (*p*TPMDS) 95% confidence interval | | | | | Morphometric Vitality Index (*c*MVI) 95% confidence interval | | | | | Developmental Disability Index (*p*DDI) 95% confidence interval | | | | |
|---|---|---|---|---|---|---|---|---|---|---|---|---|---|---|---|
| | N | Odds ratio | Lower limit | Upper limit | *P* value | N | Odds ratio | Lower limit | Upper limit | *P* value | N | Odds ratio | Lower limit | Upper limit | *P* value |
| PIVH grade 3 | 5,201 | 5.82 | 3.01 | 11.01 | .000 | 5,280 | 3.69 | 2.13 | 6.39 | .000 | 5,197 | 9.58 | 4.40 | 20.82 | .000 |
| PIVH grade 4 | 5,202 | 7.25 | 2.55 | 20.59 | .000 | 5,281 | 15.98 | 3.83 | 66.62 | .000 | 5,197 | 9.67 | 2.95 | 31.69 | .000 |
| PIVH present (all grades) | 5,202 | 6.42 | 5.46 | 13.79 | .000 | 5,281 | 4.52 | 3.25 | 6.28 | .000 | 5,198 | 5.88 | 4.01 | 8.47 | .000 |
| WMD present | 5,202 | 8.65 | 5.46 | 13.70 | .000 | 5,281 | 5.96 | 4.01 | 8.86 | .000 | 5,198 | 191.20 | 26.79 | 1361.86 | .000 |
| PIVH plus WMD vs PIVH only | 230 | 9.21 | 3.75 | 22.60 | .000 | 232 | 8.57 | 4.00 | 18.38 | .000 | 227 | 105.96 | 14.08 | 797.20 | .000 |
| PIVH without WMD | 5,050 | 2.41 | 1.48 | 3.91 | .000 | 5,050 | 1.61 | 1.02 | 2.54 | .052 | 5,048 | 1.50 | 0.95 | 2.37 | .085 |
| PIVH grade 1+2 (exclusive) | 4,973 | 1.82 | 0.98 | 3.38 | .065 | 5,049 | 0.93 | 0.51 | 1.69 | .879 | 4,970 | 0.94 | 0.52 | 1.72 | .879 |
| Breech presentation | 5,198 | 3.62 | 2.45 | 4.60 | .000 | 5,277 | 2.95 | 2.35 | 3.69 | .000 | 5,194 | 1.74 | 1.42 | 2.14 | .000 |
| Breech presentation, vag. delivery | 374 | 0.61 | 0.48 | 0.77 | .000 | 379 | 0.76 | 0.60 | 0.97 | .042 | 373 | 0.45 | 0.43 | 0.69 | .000 |
| Cardiotocography pathologic | 5,202 | 2.99 | 2.53 | 3.45 | .000 | 5,281 | 2.12 | 1.81 | 2.47 | .000 | 5,198 | 1.42 | 1.23 | 1.65 | .000 |
| sex | 5,196 | 1.10 | 1.04 | 1.16 | .001 | 5,275 | 1.27 | 1.20 | 1.35 | .000 | 5,192 | 1.16 | 1.10 | 1.23 | .000 |

*Jensen Newborns' growth, obstetrical risk, and psychomotor development. Am J Obstet Gynecol Glob Rep 2023.* (continued)

Table 1

| TABLE 1 | | | | | | | | | | | | | | |
|---|---|---|---|---|---|---|---|---|---|---|---|---|---|---|
| Odds ratios and 95% confidence intervals of predicted Total psychomotor development score (*pTPMDS*), *calculated* Morphometric vitality index (*cMVI*), and *predicted* Developmental disability Index (*pDDI*) for obstetrical risk factors in 5,301 newborns (24-43 weeks gestation, 350-5,370 g birthweight) derived from a cranial ultrasound screening data base[1,2,12] *(continued)* | | | | | | | | | | | | | | |
| Variable | Psychomotor Development (*pTPMDS*) 95% confidence interval | | | | | Morphometric Vitality Index (*cMVI*) 95% confidence interval | | | | | Developmental Disability Index (*pDDI*) 95% confidence interval | | | | |
| | N | Odds ratio | Lower limit | Upper limit | *P* value | N | Odds ratio | Lower limit | Upper limit | *P* value | N | Odds ratio | Lower limit | Upper limit | *P* value |
| Amnion infection | 5,199 | 1.00 | 1.00 | 1.00 | .016 | 5,278 | 1.00 | 1.00 | 1.00 | .016 | 5,195 | 5.01 | 0.59 | 42.97 | .125 |
| Bleeding, vaginal | 5,199 | 1.98 | 1.49 | 2.63 | .000 | 5,278 | 1.62 | 1.23 | 2.13 | .001 | 5,195 | 1.44 | 1.09 | 1.89 | .009 |
| Hypertension | 5,185 | 1.66 | 1.19 | 2.31 | .003 | 5,264 | 1.25 | 0.91 | 1.72 | .099 | 5,181 | 1.40 | 1.01 | 1.94 | .049 |
| Prolonged or arrested labour | 5,202 | 1.65 | 1.39 | 1.97 | .000 | 5,281 | 2.03 | 1.70 | 2.43 | .000 | 5,198 | 5.31 | 4.27 | 6.59 | .000 |
| Primiparity | 5,201 | 1.64 | 1.47 | 1.84 | .000 | 5,280 | 1.65 | 1.48 | 1.84 | .000 | 5,197 | 1.40 | 1.25 | 1.56 | .000 |
| Maternal age <3% centile | 5,183 | 2.08 | 1.42 | 3.05 | .000 | 5,262 | 2.39 | 1.62 | 3.53 | .000 | 5,179 | 2.08 | 1.42 | 3.05 | .000 |
| Transfer to NICU | 2,655 | 1.70 | 1.39 | 2.08 | .000 | 2,669 | 1.54 | 1.26 | 1.88 | .000 | 2,651 | 1.20 | 1.51 | 2.32 | .000 |
| Malformation | 5,202 | 1.80 | 0.83 | 3.89 | .184 | 5,281 | 0.38 | 0.17 | 0.86 | .024 | 5,198 | 0.40 | 0.18 | 0.91 | .035 |
| Meconium stained amniotic fluid | 5,201 | 1.39 | 1.07 | 1.81 | .015 | 5,280 | 1.76 | 1.35 | 2.29 | .000 | 5,197 | 1.80 | 1.37 | 2.35 | .000 |
| PROM | 5,202 | 1.65 | 1.44 | 1.87 | .000 | 5,281 | 1.66 | 1.50 | 1.89 | .000 | 5,198 | 1.37 | 1.21 | 1.56 | .000 |
| EPH syndrome | 5,202 | 1.63 | 1.33 | 1.99 | .000 | 5,281 | 1.40 | 1.13 | 1.66 | .002 | 5,198 | 1.28 | 1.05 | 1.55 | .016 |
| Miscarrage | 5,201 | 1.22 | 1.06 | 1.40 | .005 | 5,280 | 1.15 | 1.00 | 1.32 | .045 | 5,197 | 1.16 | 1.01 | 1.33 | .037 |
| Maternal fever >38°C | 5,202 | 1.39 | 0.76 | 2.54 | .179 | 5,281 | 1.44 | 0.79 | 2.63 | .145 | 5,198 | 0.95 | 0.52 | 1.74 | .999 |
| Rh incompatibility | 5,202 | 1.40 | 0.62 | 3.15 | .270 | 5,281 | 0.67 | 0.30 | 1.49 | .423 | 5,198 | 0.60 | 0.26 | 1.37 | .306 |

(continued)

**TABLE 1**

**Odds ratios and 95% confidence intervals of predicted Total psychomotor development score (*p*TPMDS), *calculated* Morphometric vitality index (*c*MVI), and *predicted* Developmental disability Index (*p*DDI) for obstetrical risk factors in 5,301 newborns (24-43 weeks gestation, 350-5,370 g birthweight) derived from a cranial ultrasound screening data base[1,2,12]** *(continued)*

| Variable | Psychomotor Development (*p*TPMDS) 95% confidence interval | | | | | Morphometric Vitality Index (*c*MVI) 95% confidence interval | | | | | Developmental Disability Index (*p*DDI) 95% confidence interval | | | | |
|---|---|---|---|---|---|---|---|---|---|---|---|---|---|---|---|
| | N | Odds ratio | Lower limit | Upper limit | *P* value | N | Odds ratio | Lower limit | Upper limit | *P* value | N | Odds ratio | Lower limit | Upper limit | *P* value |
| Diabetes mellitus | 5,201 | 1.10 | 0.67 | 1.81 | .706 | 5,280 | 1.13 | 0.70 | 1.84 | .706 | 5,197 | 1.07 | 0.65 | 1.76 | .800 |
| Maternal age >97% centile | 5,183 | 1.07 | 0.74 | 1.55 | .778 | 5,262 | 1.01 | 1.00 | 1.01 | .265 | 5,179 | 1.00 | 1.00 | 1.01 | .398 |
| Hypotension | 5,047 | 0.51 | 0.17 | 1.48 | .301 | 5,122 | 0.88 | 0.32 | 2,43 | .504 | 5,043 | 0.67 | 0.24 | 1.89 | .607 |

*EPH,* edema-proteinuria-hypertension; *IUGR;* intrauterine growth restriction; *MCU.* neonatal intensive care unit; *PIVH,* peri/-intraventricular hemorrhage; *PROM,* premature rupture of membranes, *WMD,* white matter brain damage.

*Jensen.. Newborns' growth, obstetrical risk, and psychomotor development. Am J Obstet Gynecol Glob Rep 2023.*

Table 2

**TABLE 2**

Multivariate analysis variance, F test, and effect size of *predicted* Intelligence quotient (*p*IQ), *predicted* Maze test results (*p*MT), and *predicted* Neurologic examination optimality score (*p*NOS) for obstetrical risk factors in 5,301 newborns (24-43 weeks gestation, 350-5,370g birthweight) derived from a cranial ultrasound screening data base[1,2,12]

| Variable | N | df | Intelligence quotient (z*p*IQ) | | | | Maze test (z*p*MT) | | | | Neurological examination optimality score (*p*zNOS) | | | |
| | | | | | Multivariate test | | | | Multivariate test | | | | Multivariate test | |
| | | | n | F test | Effect size | P value | n | F test | Effect size | P value | n | F test | Effect size | P value |
|---|---|---|---|---|---|---|---|---|---|---|---|---|---|---|
| Gestational age (centile) | 5,202 | 4 | 5,202 | 252.8 | 0.16 | .000 | 5,202 | 990.0 | 0.43 | .000 | 5,202 | 642.0 | 0.33 | .000 |
| Brain body weight ratio (centile) | 5,202 | 4 | 5,202 | 925.2 | 0.42 | .000 | 5,202 | 1525.8 | 0.54 | .000 | 5,202 | 1,142.0 | 0.47 | .000 |
| Preterm birth ≤36 weeks | 5,202 | 1 | 560 | 760.7 | 0.13 | .000 | 560 | 2629.1 | 0.34 | .000 | 560 | 1818.7 | 0.26 | .000 |
| Weight/length ratio (centile) | 5,202 | 4 | 5,202 | 616.5 | 0.32 | .000 | 5,202 | 1516.9 | 0.54 | .000 | 5,202 | 1,254.8 | 0.49 | .000 |
| IUGR | 5,202 | 1 | 187 | 375.9 | 0.07 | .000 | 187 | 26.2 | 0.01 | .000 | 187 | 402.1 | 0.07 | .000 |
| Multiples | 5,202 | 1 | 250 | 252.7 | 0.05 | .000 | 250 | 542.9 | 0.09 | .000 | 250 | 222.6 | 0.04 | .000 |
| Apgar 1 minute < 9 | 5,195 | 1 | 1,254 | 836.6 | 0.14 | .000 | 1,254 | 1151.6 | 0.18 | .000 | 1,254 | 532.7 | 0.09 | .000 |
| Apgar 5 minutes < 10 | 5,193 | 1 | 943 | 881.0 | 0.14 | .000 | 943 | 1326.2 | 0.20 | .000 | 943 | 774.5 | 0.13 | .000 |
| Apgar 10 minutes < 10 | 5,190 | 1 | 466 | 887.8 | 0.15 | .000 | 466 | 1768.0 | 0.25 | .000 | 466 | 1,266.9 | 0.20 | .000 |
| pH umbilical artery <7.29 vs.>=7.29 | 5,202 | 1 | 2,566 | 866.9 | 0.14 | .000 | 2,566 | 549.6 | 0.10 | .021 | 2,566 | 151.4 | 0.03 | .000 |
| PIVH grade 1+2 | 5,201 | 1 | 177 | 292.4 | 0.05 | .000 | 177 | 1339.6 | 0.20 | .000 | 177 | 1736.9 | 0.25 | .000 |
| PIVH grade 3 | 5,201 | 1 | 75 | 67.4 | 0.01 | .000 | 75 | 355.4 | 0.06 | .000 | 75 | 263.7 | 0.05 | .000 |
| PIVH grade 4 | 5,201 | 1 | 33 | 49.9 | 0.01 | .000 | 33 | 286.0 | 0.05 | .000 | 33 | 312.1 | 0.06 | .000 |
| PIVH present (all grades) | 5,202 | 1 | 230 | 272.5 | 0.05 | .000 | 230 | 1606.4 | 0.24 | .000 | 230 | 1,592.3 | 0.23 | .000 |
| WMD present | 5,201 | 1 | 193 | 317.4 | 0.06 | .000 | 193 | 1049.5 | 0.17 | .000 | 193 | 1,968.5 | 0.27 | .000 |

EP 4 631 419 A1

**TABLE 2**

**Multivariate analysis variance, F test, and effect size of *predicted* Intelligence quotient (*p*IQ), *predicted* Maze test results (*p*MT), and *predicted* Neurologic examination optimality score (*p*NOS) for obstetrical risk factors in 5,301 newborns (24-43 weeks gestation, 350-5,370g birthweight) derived from a cranial ultrasound screening data base[1,2,12]**

| Variable | N | df | Intelligence quotient (z*p*IQ) | | | | Maze test (z*p*MT) | | | | Neurological examination optimality score (*p*zNOS) | | | |
| | | | Multivariate test | | | | Multivariate test | | | | Multivariate test | | | |
| | | | n | F test | Effect size | *P* value | n | F test | Effect size | *P* value | n | F test | Effect size | *P* value |
|---|---|---|---|---|---|---|---|---|---|---|---|---|---|---|
| PIVH without WMD | 5,050 | 1 | 78 | 9.8 | 000 | .002 | 78 | 275.0 | .000 | .000 | 78 | 79.9 | 0.02 | .000 |
| PIVH grade 1+2 (exclusive) | 4,973 | 1 | 43 | 1.0 | 0.00 | 309 | 43 | 62.6 | 0.01 | .000 | 43 | 37.3 | 0.01 | .000 |
| Breech presentation | 5,198 | 1 | 374 | 303.0 | 0.06 | .000 | 374 | 346.5 | 0.06 | .000 | 374 | 42.1 | 0.01 | .000 |
| Breech presentation, vaginal delivery | 374 | 1 | 154 | 7.2 | 0.02 | .007 | 154 | 27.0 | 0.07 | .000 | 154 | 75.0 | 0.17 | .000 |
| Cardiotocography pathologic | 5,202 | 1 | 655 | 471.1 | 0.08 | .000 | 655 | 405.9 | 0.07 | .000 | 655 | 775.5 | 0.13 | .000 |
| Amnion infection | 5,199 | 1 | 6 | 8.0 | 0.00 | .005 | 6 | 48.9 | 0.01 | .000 | 6 | 46.0 | 0.01 | .000 |
| Bleeding, vaginal | 5,199 | 1 | 222 | 12.3 | 0.00 | .000 | 222 | 48.7 | 0.01 | .000 | 222 | 18.0 | 0.00 | .000 |
| Hypertension | 5,185 | 1 | 153 | 36.0 | 0.01 | .000 | 153 | 18.6 | 0.00 | .000 | 153 | 28.7 | 0.01 | .000 |
| Prolonged or arrested labour | 5,202 | 1 | 597 | 3.8 | 0.00 | .051 | 597 | 11.1 | 0.00 | .000 | 597 | 466.9 | 0.08 | .000 |
| Primiparity | 5,199 | 1 | 2,539 | 84.7 | 0.02 | .000 | 2,539 | 100.6 | 0.02 | .000 | 2,539 | 10.3 | 0.00 | .001 |

*Jensen. Newborns' growth, obstetrical risk, and psychomotor development. Am J Obstet Gynecol Glob Rep 2023.*                    *(continued)*

EP 4 631 419 A1

Table 2

**TABLE 2**
**Multivariate analysis variance, F test, and effect size of** *predicted* **Intelligence quotient (**pIQ**),** *predicted* **Maze test results (**pMT**), and** *predicted* **Neurologic examination optimality score (**pNOS**) for obstetrical risk factors in 5,301 newborns (24-43 weeks gestation, 350-5,370g birthweight) derived from a cranial ultrasound screening data base[1,2,12]** *(continued)*

| Variable | N | df | n | F test | Intelligence quotient (zpIQ) Multivariate test Effect size | P value | n | F test | Maze test (zpMT) Multivariate test Effect size | P value | n | F test | Neurological examination optimality score (pzNOS) Multivariate test Effect size | P value |
|---|---|---|---|---|---|---|---|---|---|---|---|---|---|---|
| Maternal age <3 percentile | 5,183 | 1 | 123 | 0.6 | 0.00 | .432 | 123 | 5.1 | 0.00 | 024 | 123 | 12.3 | 0.00 | .000 |
| Transfer to NICU | 2,655 | 1 | 353 | 68.1 | 0.03 | .000 | 353 | 26.4 | 0.01 | .000 | 353 | 30.2 | 0.01 | .000 |
| Malformation | 5,202 | 1 | 28 | 18.9 | 0.00 | .000 | 28 | 14.3 | 0.00 | .000 | 28 | 18.1 | 0.00 | .000 |
| Meconium stained amniotic fluid | 5,201 | 1 | 242 | 3.2 | 0.00 | .073 | 242 | 16.9 | 0.00 | .000 | 242 | 4.0 | 0.00 | .046 |
| PROM | 5,202 | 1 | 829 | 19.9 | 0.00 | .000 | 829 | 97.0 | 0.02 | .000 | 829 | 34.4 | 0.01 | .000 |
| EPH syndrome | 5,202 | 1 | 378 | 93.4 | 0.02 | .000 | 378 | 47.1 | 0.01 | .000 | 378 | 61.8 | 0.01 | .000 |
| Miscarrage | 5,201 | 1 | 1,029 | 6.0 | 0.00 | .015 | 1,029 | 25.6 | 0.00 | .000 | 1,029 | 7.5 | 0.00 | .006 |
| sex | 5,196 | 1 | 2,529 | 10.2 | 0.00 | .001 | 2,529 | 20.3 | 0.00 | .000 | 2,529 | 16.4 | 0.00 | .000 |
| Maternal fever >38°C | 5,202 | 1 | 43 | 0.0 | 0.00 | .974 | 43 | 4.6 | 0.00 | .031 | 43 | 5.2 | 0.00 | .023 |
| Rh incompatibility | 5,202 | 1 | 24 | 0.1 | 0.00 | .821 | 24 | 1.3 | 0.00 | .257 | 24 | 5.7 | 0.00 | .017 |
| Diabetes mellitus | 5,201 | 1 | 63 | 1.6 | 0.00 | .205 | 63 | 5.1 | 0.00 | .024 | 63 | 0.1 | 0.00 | .745 |
| Maternal age >97 percentile | 5,183 | 1 | 116 | 1.1 | 0.00 | .287 | 116 | 0.4 | 0.00 | .543 | 116 | 0.5 | 0.00 | .914 |
| Hypotension | 5,047 | 1 | 15 | 0.3 | 0.00 | .582 | 15 | 0.3 | 0.00 | .565 | 15 | 0.0 | 0.00 | .938 |

*EPH,* edema-proteinuria-hypertension; *IUGR,* intrauterine growth restriction; *NICU,* neonatal intensive care unit; *PIVH,* hemorrhage; *PROM,* premature rupture of membranes; *WMD,* white matter brain damage.

Jensen. *Newborns' growth,* obstetrical *risk, and psychomotor development. Am J Obstet Gynecol Glob Rep 2023.*

**Claims**

1. A method for predicting a risk of brain damage in a newborn and/or a physical state of the newborn prior to birth of the newborn during pregnancy, the method comprising the following steps:

   i) Determining the presence of one or more risk factors during pregnancy which are associated with brain damage in the newborn;
   ii) Calculating a pregnancy risk score by summing up odds ratios which quantify the association between the one or more risk factors and a poor predicted psychomotor development of infants at preschool age;
   iii) Using a regression equation to predict a risk of brain damage in the newborn and/or the physical state of the newborn based on the calculated pregnancy risk score.

2. The method of claim 1, wherein the one or more risk factors are chosen from a list consisting of gestational age, brain body weight ratio, weight/head circumference ratio, preterm birth $\leq$36 wk, weight/length ratio, IUGR, multiples, Apgar 1 min, score <9, Apgar 1 min, score <7, Apgar 5 min, score <10, Apgar 5 min. score <9, Apgar 10 min. score <10, Apgar 10 min, score <9, pH umbilical artery <7.29 vs. $\geq$7.29, PIVH grade 1+2, PIVH grade 3, PIVH grade 4, PIVH present (all grades), WMD present, PIVH plus WMD vs PIVH only, PIVH without WMD, PIVH grade 1+2 (exclusive), presentation, vaginal delivery, cardiotocography pathologic, sex, amnion infection, vaginal bleeding, hypertension, prolonged or arrested labor, primiparity, maternal age <3% centile, transfer to NICU, malformation, meconium stained amniotic fluid, PROM, EPH syndrome, miscarriage, maternal fever >38°C, Rh incompatibility, diabetes mellitus, maternal age >97% centile and hypotension.

3. The method of any of the proceeding claims, wherein the regression equation in step iii) is a linear regression equation which contains a constant and a regression coefficient, wherein the constant and the regression coefficient have been calculated using a linear regression analysis.

4. The method according to claim 3, wherein in step iii) the following linear regression equation is used to calculate the risk of brain damage as a dependent variable:

$$pBrain\_damage = b_1 + m_1 * pregnancy\ risk\ score,$$

   wherein $b_1$ is chosen from the range of 0.0 to 0.1, preferably of 0.0 to 0.05, more preferably 0.021 and, wherein $m_1$ is chosen from the range of 0.0 to 0.1, preferably 0.0 to 0.01, more preferably 0.005.

5. The method according to claim 3, wherein the physical state of the newborn is defined by an Apgar score at 1 minute (Apgar 1 min), an Apgar score at 5 minutes (Apgar 5 min) and/or an Apgar score at 10 minutes (Apgar 10 min) after birth of the newborn and, wherein in step iii) the following linear regression equation is used to calculate Apgar 1 min, Apgar 5 min and/or Apgar 10 min:

   a) $pApgar\_1 = b_2 - m_2 *$ pregnancy risk score, wherein pApgar_1 is the predicted Apgar 1 min, wherein $b_2$ is chosen from the range of 8.5 to 9, preferably 8.75 to 8.9, more preferably 8.889 and wherein $m_2$ is chosen from the range of 0.0 to 0.1, preferably 0.0 to 0.05, more preferably 0.041,
   b) $pApgar\_5 = b_3 - m_3 *$ pregnancy risk score, wherein pApgar_5 is the predicted Apgar 5 min, wherein $b_3$ is chosen from the range of 9.5 to 10, preferably 9.7 to 9.9, more preferably 9.858 and wherein $m_3$ is chosen from the range of 0.0 to 0.1, preferably 0.0 to 0.05, more preferably 0.024,
   c) $pApgar\_10 = b_4 - m_4 *$ pregnancy risk score, wherein pApgar_10 is the predicted Apgar 10 min, wherein $b_4$ is chosen from the range of 9.5 to 10, preferably 9.7 to 9.9, more preferably 9.980 and wherein $m_4$ is chosen from the range of 0.0 to 0.1, preferably 0.0 to 0.05, more preferably 0.014.

6. The method of any of the proceeding claims, wherein the risk of brain damage in the newborn and/or the physical state of the newborn based on the calculated pregnancy risk score as determined in step iii) are outputted on a display.

7. The method of any of the proceeding claims, wherein at least steps ii) and iii) are executed on a computer comprising a processor, a storage medium and an input interface to receive user inputs.

Fig. 1

Fig. 2

Fig. 3

Fig. 4

Fig. 5

Fig. 6

Fig. 7

Fig. 8

Europäisches
Patentamt

European
Patent Office

Office européen
des brevets

**EUROPEAN SEARCH REPORT**

Application Number

EP 25 16 8859

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JENSEN ARNE ET AL: "Growth variables and obstetrical risk factors in newborns are associated with psychomotor development at preschool age", AJOG GLOBAL REPORTS, [Online] vol. 3, no. 4, 100219, 8 May 2023 (2023-05-08), pages 1-13, XP093307573, ISSN: 2666-5778, DOI: 10.1016/j.xagr.2023.100219 Retrieved from the Internet: URL:https://www.sciencedirect.com/science/article/pii/S2666577823000606/pdfft?md5=e1c49398743747fb6d155c3d03ca1ee1&pid=1-s2.0-S2666577823000606-main.pdf> [retrieved on 2025-08-26] * abstract * * sect."Materials and Methods"; pages 2,7 * * page 2, column center - right-hand column * * tables 1,2 * ----- -/-- | 1-7 | INV. A61B5/00 G16H50/20 G16H50/30 |
| | | | TECHNICAL FIELDS SEARCHED (IPC) A61B G16H |

The present search report has been drawn up for all claims

| Place of search | Date of completion of the search | Examiner |
|---|---|---|
| The Hague | 27 August 2025 | Delval, Christophe |

EPO FORM 1503 03.82 (P04C01)

page 1 of 4

Europäisches
Patentamt

European
Patent Office

Office européen
des brevets

# EUROPEAN SEARCH REPORT

**Application Number**

EP 25 16 8859

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JENSEN ARNE ET AL: "Differential effects of growth restriction and immaturity on predicted psychomotor development at 4 years of age in preterm infants", AJOG GLOBAL REPORTS, [Online] vol. 4, no. 1, 100305, 9 January 2024 (2024-01-09), pages 1-18, XP093307595, ISSN: 2666-5778, DOI: 10.1016/j.xagr.2023.100305 Retrieved from the Internet: URL:https://www.sciencedirect.com/science/article/pii/S2666577823001478/pdfft?md5=df8ffefb3bd9c949b4d89996287949c9&pid=1-s2.0-S2666577823001478-main.pdf> [retrieved on 2025-08-26] * abstract * * sect."Materials and Methods" * * figures 1-3; tables 1-5 * ----- -/-- | 1-7 | |
| | | | **TECHNICAL FIELDS SEARCHED (IPC)** |

The present search report has been drawn up for all claims

| Place of search | Date of completion of the search | Examiner |
|---|---|---|
| The Hague | 27 August 2025 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt

European
Patent Office

Office européen
des brevets

# EUROPEAN SEARCH REPORT

**Application Number**

EP 25 16 8859

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WU MINGYANG ET AL: "Association between early-term birth and delayed neurodevelopment at the age of 2 years: results from a cohort study in China", EUROPEAN JOURNAL OF PEDIATRICS, SPRINGER BERLIN HEIDELBERG, BERLIN/HEIDELBERG, [Online] vol. 180, no. 12, 17 June 2021 (2021-06-17), pages 3509-3517, XP037615767, ISSN: 0340-6199, DOI: 10.1007/S00431-021-04152-6 Retrieved from the Internet: URL:https://link.springer.com/content/pdf/ 10.1007/s00431-021-04152-6.pdf> [retrieved on 2021-06-17] * abstract * * sect."Outcome measures" * * sect."Statistical analysis" * * tables 2-4 * ----- | 1-7 | |
| X | US 2024/038402 A1 (PERI VENKAT NARASIMHAM [US] ET AL) 1 February 2024 (2024-02-01) * abstract * * paragraphs [0034], [0103] - [0154], [0168] - [0170], [0439]; figure 3 * ----- | 1-7 | **TECHNICAL FIELDS SEARCHED (IPC)** |

-/--

The present search report has been drawn up for all claims

| Place of search | Date of completion of the search | Examiner |
|---|---|---|
| The Hague | 27 August 2025 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 16 8859

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JENSEN ARNE ET AL: "Association of weight-length ratio at birth with psychomotor trajectories among preschool-aged children", AJOG GLOBAL REPORTS, [Online] vol. 2, no. 4, 100115, 2 October 2022 (2022-10-02), pages 1-2, XP093307644, ISSN: 2666-5778, DOI: 10.1016/j.xagr.2022.100115 Retrieved from the Internet: URL:https://www.sciencedirect.com/science/article/pii/S2666577822000648/pdfft?md5=99d769c698cc79a54e3cbb057189ec9c&pid=1-s2.0-S2666577822000648-main.pdf> [retrieved on 2025-08-26] * abstract * * the whole document * | 1-7 | |
| A | US 2022/007999 A1 (EVANS MARK [US]) 13 January 2022 (2022-01-13) * abstract * * paragraphs [0002], [0055] - [0060], [0117] - [0128]; claims 1-11 * | 1-7 | TECHNICAL FIELDS SEARCHED (IPC) |

The present search report has been drawn up for all claims

| Place of search | Date of completion of the search | Examiner |
|---|---|---|
| The Hague | 27 August 2025 | Delval, Christophe |

EPO FORM 1503 03.82 (P04C01)

## ANNEX TO THE EUROPEAN SEARCH REPORT
## ON EUROPEAN PATENT APPLICATION NO.

EP 25 16 8859

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2025

| Patent document cited in search report | Publication date | Patent family member(s) | | Publication date |
|---|---|---|---|---|
| US 2024038402 A1 | 01-02-2024 | EP | 4069058 A1 | 12-10-2022 |
| | | US | 2021118574 A1 | 22-04-2021 |
| | | US | 2024038402 A1 | 01-02-2024 |
| | | WO | 2021079374 A1 | 29-04-2021 |
| US 2022007999 A1 | 13-01-2022 | BR | 112021009203 A2 | 03-08-2021 |
| | | CA | 3119938 A1 | 22-05-2020 |
| | | CN | 113677263 A | 19-11-2021 |
| | | EP | 3880067 A1 | 22-09-2021 |
| | | IL | 283048 A | 30-06-2021 |
| | | JP | 7579248 B2 | 07-11-2024 |
| | | JP | 2022507547 A | 18-01-2022 |
| | | US | 2022007999 A1 | 13-01-2022 |
| | | WO | 2020102524 A1 | 22-05-2020 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERGER et al.** *Eur. J. Obstet. Gynaecol. Reprod. Biol.*, 1997, vol. 75, 191-203
- **MIDDELANIS et al.** *J. Soc. Gynecol. Investig.*, 2003, vol. 10 (2)
- **A. JENSEN**. Stammzellen aus Nabelschnurblut heilen kindlichen Hirnschaden" [Stem cells from cord blood heal infant brain damage. *Top Magazine Ruhr, Wissenschaft - Medizin [Science - Medicine*, 2009, vol. 23 (4), 80-81
- **A. JENSEN**. Erste Therapie eines kindlichen hypoxischen Hirnschadens mit Zerebralparese nach Herzstillstand? [First therapy of hypoxic infant brain damage with cerebral paresis after cardiac arrest?. *Heilversuch durch autologe Nabelschnurstammzell-Transplantation." [attempt at healing through autologous cord stem cell transplantation] Regenerative Medizin [Medicine*, 2011, vol. 4 (1), 30-31
- **A. JENSEN et al.** Perinatal brain damage - from neuroprotection to neuro regeneration using cord blood stem cells. *Med. Klein.*, 2003, vol. 98 (2), 22-26
- **C. MEIER et al.** Spastic paresis after perinatal brain damage in rats is reduced by human cord blood mononuclear cells. *Pediatr. Res.*, 2006, vol. 59 (2), 244-249
- **A. JENSEN**. Growth variables and obstetrical risk factors in newborns are associated with psychomotor development at preschool age. *AJOG Global Reports*, 08 May 2023, vol. 3 (4), 100219